# EUROPEAN PATENT SPECIFICATION

(11) **EP 4 061 152 B1**
(45) Date of publication and mention of the grant of the patent: **01.11.2023**
(21) Application number: 20810967.8
(22) Date of filing: 23.11.2020
(51) Int. Cl.: A23L 33/12, A23L 33/00

(54) **INFANT FORMULA FOR REDUCING THE RISK OF DEVELOPING NON-ALCOHOLIC FATTY LIVER DISEASE**
SÄUGLINGSNAHRUNG ZUR VERRINGERUNG DES RISIKOS DER ENTWICKLUNG VON NICHTALKOHOLISCHER FETTLEBERERKRANKUNG
FORMULE POUR NOURRISSON PERMETTANT DE RÉDUIRE LE RISQUE DE DÉVELOPPER UNE STÉATOSE HÉPATIQUE D'ORIGINE NON ALCOOLIQUE

(30) Priority: 22.11.2019 EP 19211021
(43) Date of publication of application: 28.09.2022
(73) Proprietor: N.V. Nutricia, 2712 HM Zoetermeer (NL)
(72) Inventor: KODDE, Francina Dorothea, 3584 CT Utrecht (NL); RAKHSHANDEHROO, Maryam, 3584 CT Utrecht (NL); VAN DER BEEK, Eline Marleen, 3584 CT Utrecht (NL); OOSTERVEER, Maaike Hélène, 3584 CT Utrecht (NL)
(74) Representative: Nederlandsch Octrooibureau
(86) International application number: PCT/EP2020/083047
(87) International publication number: WO 2021/099632

(56) References cited:
- WO-A1-2010/027258
- NOBILI VALERIO ET AL: "Breastfeeding and NAFLD from the maternal side of the mother-infant dyad", JOURNAL OF HEPATOLOGY, vol. 70, no. 1, 30 October 2018 (2018-10-30), pages 13-14, XP085558907, ISSN: 0168-8278, DOI: 10.1016/J.JHEP.2018.10.030
- MOURALIDARANE A ET AL: "Maternal obesity programs offspring non-alcoholic fatty liver disease through disruption of 24-h rhythms in mice", INTERNATIONAL JOURNAL OF OBESITY, NEWMAN PUBLISHING, LONDON, GB, vol. 39, no. 9, 14 May 2015 (2015-05-14), pages 1339-1348, XP036971876, ISSN: 0307-0565, DOI: 10.1038/IJO.2015.85 [retrieved on 2015-05-14]
- ZHOU YI ET AL: "Sex-associated preventive effects of low-dose aspirin on obesity and non-alcoholic fatty liver disease in mouse offspring with over-nutrition in utero", LABORATORY INVESTIGATION, NATURE PUBLISHING GROUP, THE UNITED STATES AND CANADIAN ACADEMY OF PATHOLOGY, INC, vol. 99, no. 2, 9 November 2018 (2018-11-09), pages 244-259, XP036846881, ISSN: 0023-6837, DOI: 10.1038/S41374-018-0144-2 [retrieved on 2018-11-09]
- ZHANG LI ET AL: "Increased susceptibility of prenatal food restricted offspring to high-fat diet-induced nonalcoholic fatty liver disease is intrauterine programmed", REPRODUCTIVE TOXICOLOGY, ELSEVIER SCIENCE, US, vol. 65, 16 August 2016 (2016-08-16), pages 236-247, XP029765290, ISSN: 0890-6238, DOI: 10.1016/J.REPROTOX.2016.08.006

## Description

### FIELD OF THE INVENTION

The invention relates to a nutritional composition for infants, in particular infant formula, follow-on formula or growing-up milk for use in reducing the risk of developing non-alcoholic fatty liver disease in infants at risk of developing non-alcoholic fatty liver disease.

### BACKGROUND OF THE INVENTION

Human milk is the uncontested gold standard concerning infant nutrition. However, in some cases breastfeeding is inadequate or unsuccessful for medical reasons or because of a choice not to breastfeed. For such situations infant or follow-on formulas have been developed. Commercial infant formulas are commonly used today to provide supplemental or sole source of nutrition early in life. These formulas comprise a range of nutrients to meet the nutritional needs of the growing infant, and typically include fat, carbohydrate, protein, vitamins, minerals, and other nutrients helpful for optimal infant growth and development. Commercial infant formulas are designed to mimic, as closely as possible, the composition and function of human milk.

Human milk lipids are known to have a distinct physical structure composed of large lipid globules with an average mode diameter of about 4 µm existing of a triglyceride core coated by a tri-layer of membranes, the milk fat globule membrane (MFGM). The mode diameter of lipid droplets in standard infant formula is typically about 0.3-0.5 µm due to the industrial processing procedures to achieve stable and reproducible end products, and the lipid droplets are not surrounded by MFGM but mostly by milk proteins. Infant formula with lipid globules with an architecture more similar to the lipid globules in human milk have been described.

Non-alcoholic fatty liver disease (NAFLD) is increasingly common around the world, especially in Western nations. In the United States, it is the most common form of chronic liver disease, affecting about one-quarter of the population. While detailed descriptions of NAFLD in adults can be found in the literature as far back as the 1850s, the first case of paediatric NAFLD was reported in 1983. It is now the most prevalent form of chronic liver disease in childhood and adolescence, affecting approximately 10%-20% of the general paediatric population in the Western World.

NAFLD is an umbrella term for a range of liver conditions affecting people who drink little to no alcohol. As the name implies, the main characteristic of NAFLD is too much fat stored in liver cells. Isolated steatosis, defined by abnormal accumulation of fat in more than 5% of hepatocytes, is a relatively benign condition. Some individuals with NAFLD can develop non-alcoholic steatohepatitis (NASH), an aggressive form of fatty liver disease, which is marked by liver inflammation and may progress to advanced scarring (cirrhosis) and liver failure.

NAFLD is thought to be a hepatic manifestation of more widespread and underlying metabolic dysfunction and is strongly associated with a number of metabolic risk factors, including insulin resistance, dyslipidaemia, cardiovascular disease and obesity. The understanding of the pathophysiological mechanisms underpinning these relationships, however, remains incomplete. For example, an obese person does not always develop NAFLD and vice versa, a person with NAFLD is not always overweight or obese.

The clinical discovery of paediatric NAFLD has led to speculation that the origin of paediatric NAFLD may lie in early intrauterine exposures. There are indications that human infants born to obese and/or gestational diabetic mothers have increased liver fat compared with infants of normal mothers.

Brumbaugh et al. [J Pediatr. 2013, 162(5), 930-936] describes a study design wherein 25 neonates, born to normal weight mothers (n=13) and to obese mothers with gestational diabetes (GDM) (n=12), underwent MRI for measurement of subcutaneous and intra-abdominal fat and magnetic resonance spectroscopy for the measurement of intrahepatocellular (IHCL) fat at 1-3 weeks of age. The infants born to obese/GDM mothers had a mean 68% increase in IHCL compared with infants born to normal weight mothers. For all infants, IHCL correlated with maternal pre-pregnancy BMI but not with subcutaneous adiposity.

Ayonrinde et al. [J Hepatol. 2017, 67(3), 568-576] examined the association of maternal factors and infant nutrition, with the subsequent diagnosis of NAFLD in adolescents in the Western Australian Pregnancy (Raine) Cohort study. NAFLD was diagnosed in 15.2% of the 1,170 adolescents examined. Ninety-four percent had been breastfed as infants. The duration of breastfeeding before starting supplementary milk was *≥*4 months in 54.4% and *≥*6 months in 40.6%. Breastfeeding without supplementary milk ≥6 months, maternal pre-pregnancy obesity and adolescent obesity were associated with NAFLD independent of a Western dietary pattern at 17 years of age. Adolescents with NAFLD who had been breastfed for >6 months had a less adverse metabolic profile compared with adolescents breastfed for <6 months. Supplementary milk intake starting before 6 months was associated with a higher prevalence and ultrasound severity of NAFLD compared with intake starting after 6 months.

It is suggested that non-alcoholic fatty liver disease is programmed in the offspring of mothers with metabolic health challenges during pregnancy such as obesity and GDM. In the presence of such challenges, the gestational environment drives an increased metabolic fuel delivery to the fetus, leading to fetal hepatic fat accumulation, immune cell priming, epigenetic changes (first hit). This fetal liver is now "primed" for postnatal fat storage and inflammation. An obesogenic environment later in life (second hit) triggers further steatosis, hepatocyte injury, inflammation, and fibrosis which triggers progression of steatosis to steatohepatitis and eventually cirrhosis. As described in Brumbaugh et al. Pediatr Res. 2014, 75(0), 140-147.

Bruce et al. [Hepatology 2009, 50(6),1796-808] describes a study wherein female mice were fed either a high-fat (HF) or control chow (C) diet before and during gestation and lactation. Resulting offspring were fed either a C or a HF diet after weaning, to generate four offspring groups; HF/HF, HF/C, C/HF, C/C. At 15 weeks of age, liver histology was normal in both the C/C and HF/C offspring. Kleiner scoring showed that although the C/HF offspring developed non-alcoholic fatty liver disease, the HF/HF offspring developed non-alcoholic steatohepatitis (NASH). At 30 weeks, histological analysis and Kleiner scoring showed that both the HF/C and C/HF groups had NAFLD, whereas the HF/HF had a more severe form of NASH. So, even offspring which was only exposed to HF chow during pregnancy and lactation, and from weaning is only fed a control chow, develops NAFLD at 30 weeks.

Thorn et al. [Diabetes 2014, 63, 2702-2713] describes a study wherein they examined the maternal response to chronic maternal high-fat (HF) diet and the impact of post weaning healthy diet on mechanisms for NAFLD development in juvenile nonhuman primate (NHP) offspring at 1 year of age. Pregnant females on HF diet were segregated as insulin resistant (IR; HF+IR) or insulin sensitive (IS; HF+IS) compared with control (CON)-fed mothers. HF+IR mothers have increased body mass, higher triglycerides, and increased placental cytokines. At weaning, offspring were placed on a CON or HF diet. Only offspring from HF+IR mothers had increased liver triglycerides and upregulated pathways for hepatic de novo lipid synthesis and inflammation that was irreversible upon switching to a healthy diet. Thorn et al. observes that unlike juvenile offspring from HF+IS mothers, which largely resembled CON offspring, offspring from HF+IR mothers showed a pattern of hepatic gene expression, cytokines, and lipogenic responses consistent with the early stages of NAFLD. In addition, Thorn et al. observed that the NAFLD phenotype persisted despite the absence of postnatal obesity, insulin resistance, or systemic or local adipose tissue inflammation. Thorn et al. concludes that these findings indicate that maternal metabolic dysfunction programs the NAFLD phenotype and suggests that postnatal diet may be incapable of reversing or correcting this disorder in juvenile offspring.

WO 2010/0027258 and WO 2010/0027259 relate to infant formulas with large lipid globules coated with phospholipids for the prevention of obesity later in life. In the examples in these documents an observation is made about differences in liver weight, after exposure to Western-style diet later in life, between a mice group that consumed in early life a diet with large lipid globules coated with phospholipids and a mice group that consumed a control diet with small globules without phospholipid coating. It was observed that the liver weight was slightly lower in the mice group that consumed the diet with the large lipid globules with phospholipid coating in early life.

The present invention aims to provide infant nutrition beneficially affecting the risk of developing NAFLD in subjects at risk of developing NAFLD.

### SUMMARY OF THE INVENTION

The present inventors have surprisingly found that the consumption of a nutritional composition comprising large lipid globules coated with phospholipids in early life protects against excessive fat storage in the liver later in life after exposure to an obesogenic environment in a subject at risk of developing NAFLD and therefore reduces the risk of developing NAFLD later in life.

A mouse model was used, wherein a GDM-like phenotype was induced in dams by feeding them a high-fat diet (to reduce insulin sensitivity) and exposing them to three mild Streptozotocin triggers (to reduce beta cell capacity). The offspring of these GDM dams were fed in early life either with a nutritional composition according to the invention, i.e. a nutritional composition comprising large lipid globules coated with phospholipids, or a control nutritional composition comprising small lipid globules without a phospholipid coating. Offspring of GDM dams are more at risk of developing NAFLD. After exposure to a Western style diet (WSD) later in life for a long period of time, the body weight and adiposity of the offspring fed with either nutritional composition early in life were not significantly different. However, the amount of hepatic triglycerides was substantially lower in the offspring which was fed the nutritional composition according to the invention early in life, compared to the offspring that was fed the control nutritional composition. Since the energy intake was very similar for both offspring groups, it is surprising that the difference in lipid globule design (larger lipid globules with PL coating) in an IMF fed early in life has such a protective effect against excessive fat storage in the liver when challenged later in life.

This is indicative that consumption in early life of the nutritional composition according to the invention, i.e. comprising large lipid globules with a phospholipid coating, reduces the risk of developing a fatty liver later in life in a population at-risk of developing NAFLD.

The present invention therefore pertains to a nutritional composition, selected from infant formula, follow-on formula and growing-up milk, for use in reducing the risk of developing non-alcoholic fatty liver disease (NAFLD) in a human subject selected from the group consisting of:
a) an infant born to an overweight and/or obese mother at the time of conception of the infant;
b) an infant born to a mother with diabetes at the time of conception of the infant;
c) an infant born to a mother with gestational diabetes (GDM); and
d) an infant who was large for its gestational age (LGA) at birth;
said nutritional composition comprising digestible carbohydrates, protein and lipid, wherein the lipid is in the form of lipid globules and wherein:
i. the lipid globules have a mode diameter, based on volume, of at least 1.0 µm and/or at least 45 volume %, based on total lipid volume, of the lipid globules have a diameter of 2 to 12 µm; and
ii. the lipid globules are at least partly coated on the surface with phospholipids, the amount of phospholipids present in the nutritional composition being from 0.5 to 20 wt.% phospholipids based on total lipids.

### DETAILED DESCRIPTION OF THE INVENTION

In the context of the present invention, the infant formula or follow-on formula is not native cow's milk or human milk.

In the context of the present invention, growing-up milk can also be named young child formula.

In the context of the present invention a mother that is overweight or obese at the time of conception of the infant is based on the BMI of the mother before pregnancy. In one embodiment, the BMI before pregnancy is defined as the BMI as measured in the time period at least six months before conception.

In this specification, the following terms have the meanings assigned to them here below (Chiu M et al. Diabetes Care, 2011, 34:1741-1748):
The term 'BMI' as used herein, is defined as the body mass divided by the square of the body height, and is expressed in units of kg/m2. The BMI broadly categorizes a person as underweight, normal weight, overweight, or obese according to the calculated BMI value.

The terms 'underweight', 'normal weight', 'overweight' and 'obese' as herein are defined as follows: Underweight is defined as a BMI < 18.5 kg/m2 for all women. A normal weight for Asian women is considered a BMI ≥18.5 and <23 kg/m2, for non-Asian women, a normal weight is a BMI ≥18.5 and <25 kg/m2. Overweight for Asian women is considered a BMI ≥ 23 and < 27.5 kg/m2, for non-Asian women, overweight is a BMI ≥ 25 and <30 kg/m2. Obese for Asian women is considered a BMI ≥ 27.5 kg/m2, for non-Asian women, obese is a BMI ≥ 30 kg/m2.

In the context of the present invention a 'mother with diabetes at the time of conception' is a mother who has been diagnosed by a health care professional with diabetes mellitus type 1 or diabetes mellitus type 2. In the context of this invention this diagnosis of diabetes mellitus type 1 or 2 was made by a health care professional at the latest at the end of the 1^{st} trimester during the pregnancy of the infant. Preferably the mother with diabetes at the time of conception is a mother with diabetes mellitus type 2.

In the context of the present invention a 'mother with gestational diabetes' is a mother who has been diagnosed by a health care professional to suffer from gestational diabetes mellitus (GDM) during pregnancy. In the context of the present invention the GDM diagnosis is preferably based on IADPSG criteria (IADPSG: International Association of Diabetes and Pregnancy Study Group). Typically, a GDM diagnosis is made at the earliest in the second trimester during pregnancy. GDM occurs in pregnant women without a previous history of diabetes and is transient, i.e. the pathology disappears when the woman is not pregnant anymore.

In the context of the present invention 'an infant who was large for its gestational age (LGA) at birth' is an infant with a weight, length, or head circumference at birth that lies above the 90th percentile for that gestational age. Both infants born to women with pre-pregnancy overweight or obesity and infants born to women with diabetes (diabetes mellitus type 1, diabetes mellitus type 2 or GDM) have a higher risk of being LGA.

### Application

The human subject is selected from the group consisting of:
a) an infant born to an overweight and/or obese mother at the time of conception of the infant;
b) an infant born to a mother with diabetes at the time of conception of the infant;
c) an infant born to a mother with GDM; and
d) an infant who was LGA at birth.

The infants a)-d) listed here above are all considered by the skilled person to be populations at-risk of developing NAFLD later in life.

In a preferred embodiment the human subject is selected from the group consisting of:
a) an infant born to an overweight and/or obese mother at the time of conception of the infant;
b) an infant born to a mother with diabetes at the time of conception of the infant; and
c) an infant born to a mother with GDM.

In a more preferred embodiment the human subject is selected from the group consisting of:
a) an infant born to a mother with diabetes at the time of conception of the infant; and
b) an infant born to a mother with GDM.

In an even more preferred embodiment the human subject is an infant born to a mother with GDM.

In an even more preferred embodiment the human subject is an infant born to an overweight and/or obese mother at the time of conception of the infant and wherein the mother also has GDM.

In a preferred embodiment the nutritional composition is for use in reducing the risk of developing paediatric NAFLD.

In the context of the present invention the risk of developing NAFLD is reduced compared to subjects at risk of developing NAFLD who did not consume the nutritional composition, selected from infant formula, follow-on formula and growing-up milk, according to the invention. Preferably for a proper comparison the age of the subject, the type of subject [(a), b), c) or d)] and period wherein the nutritional composition is administered correspond.

In preferred embodiment of the present invention, the risk of developing NAFLD is reduced compared to the risk of developing NAFLD in a human subject selected from the group consisting of:
a) an infant born to an overweight and/or obese mother at the time of conception of the infant;
b) an infant born to a mother with diabetes at the time of conception of the infant;
c) an infant born to a mother with GDM; and
d) an infant who was LGA at birth;
wherein the subject was fed a nutritional composition, selected from infant formula, follow-on formula and growing-up milk, comprising digestible carbohydrates, protein and lipid, said nutritional composition comprising less than 0.5 wt.% phospholipids based on total lipids and having lipid globules with a mode diameter, based on volume, of about 0.5 µm.

In another embodiment of the present invention, the risk of developing NAFLD is reduced compared to the risk of developing NAFLD in a human subject selected from the group consisting of:
a) an infant born to an overweight and/or obese mother at the time of conception of the infant;
b) an infant born to a mother with diabetes at the time of conception of the infant;
c) an infant born to a mother with GDM; and
d) an infant who was LGA at birth;
wherein the subject was fed a nutritional composition, selected from infant formula, follow-on formula and growing-up milk, comprising digestible carbohydrates, protein and lipid, wherein the lipid comprises at least 10 wt.% palmitic acid based on total fatty acids, yet with less than 15 % of the palmitic acid residues being in the sn-2 position of triglycerides, said nutritional composition comprising less than 0.5 wt.% phospholipids based on total lipids and wherein the lipid globules have a mode diameter, based on volume, of about 0.5 µm.

In yet another embodiment of the present invention, the risk of developing NAFLD is reduced compared to the risk of developing NAFLD in a human subject selected from the group consisting of:
a) an infant born to an overweight and/or obese mother at the time of conception of the infant;
b) an infant born to a mother with diabetes at the time of conception of the infant;
c) an infant born to a mother with GDM; and
d) an infant who was LGA at birth;
wherein the subject was fed a nutritional composition, selected from infant formula, follow-on formula and growing-up milk, comprising digestible carbohydrates, protein and lipid, wherein the lipid comprises no mammalian milk fat, said nutritional composition comprising less than 0.5 wt.% phospholipids based on total lipids and wherein the lipid globules have a mode diameter, based on volume, of about 0.5 µm.

Thus, for these comparative nutritional compositions which are not according to the invention the volume % of lipid globules with a diameter between 2 and 12 µm is preferably below 20 vol.%, more preferably below 15 vol.% and most preferably below 10 vol.%.

These comparative nutritional compositions which are not according to the invention are preferably selected from infant formula and follow-on formula, more preferably these comparative nutritional compositions not according to the invention are infant formula.

### Lipid globule size

According to the present invention, lipid is present in the nutritional composition in the form of lipid globules. When the nutritional composition is in liquid form, these lipid globules are emulsified in the aqueous phase. Alternatively, when the nutritional composition is in powder form, the lipid globules are present in the powder and the powder is suitable for reconstitution with water or another food grade aqueous phase. The lipid globules comprise a core and a surface.

The lipid globules in the nutritional composition preferably have mode diameter, based on volume, of at least 1.0 µm, more preferably at least 3.0 µm, and most preferably at least 4.0 µm. Preferably, the lipid globules have a mode diameter, based on volume, between 1.0 and 10 µm, more preferably between 2.0 and 8.0 µm, even more preferably between 3.0 and 7.0 µm, and most preferably between 4.0 µm and 6.0 µm.

Alternatively, or preferably in addition, the size distribution of the lipid globules is preferably in such a way that at least 45 volume % (vol.%), preferably at least 55 vol.%, even more preferably at least 65 vol.%, and most preferably at least 75 vol.% of the lipid globules have a diameter between 2 and 12 µm. In a preferred embodiment, at least 45 vol.%, preferably at least 55 vol.%, more preferably at least 65 vol.%, and most preferably at least 75 vol.% of the lipid globules have a diameter between 2 and 10 µm. In a more preferred embodiment, at least 45 vol.%, more preferably at least 55 vol.%, yet even more preferably at least 65 vol.%, and most preferably at least 75 vol.% of the lipid globules have a diameter between 4 and 10 µm. Preferably less than 5 vol.% of the lipid globules have a diameter above 12 µm.

Standard infant formulae, follow-on formulae or growing-up milks have lipid globules with a mode diameter, based on volume, of below 0.5 µm. It was found that the presence of large lipid globules with a mode diameter, based on volume, of at least 1 µm, orthat a significant part, based on volume, of the lipid globules has a diameter between 2 to 12 µm, reduces the risk of developing NAFLD in an infant at risk of developing NAFLD.

The volume percentage of lipid globules is based on volume of total lipid. The mode diameter relates to the diameter which is the most present based on volume of total lipid, or the peak value in a graphic representation, having on the X-as the diameter and on the Y-as the volume %.

The volume of the lipid globule and its size distribution can suitably be determined using a particle size analyzer such as a Mastersizer (Malvern Instruments, Malvern, UK), for example by the method described in Michalski et al, 2001, Lait 81: 787-796.

### Phospholipid

The nutritional composition comprises 0.5 to 20 wt.% phospholipid based on total lipid, more preferably 0.5 to 10 wt.%, more preferably 0.75 to 8 wt.%, even more preferably 1.0 to 8 wt.%, most preferably 1.5 to 5 wt.% phospholipid based on total lipid.

Phospholipids are amphipathic of nature and include glycerophospholipids and sphingomyelin. By 'coating' is meant that the outer surface layer of the lipid globules comprises phospholipid, whereas phospholipid is virtually absent in the core of the lipid globule. A suitable way to determine whether phospholipid is located on the surface of lipid globules is confocal laser scanning microscopy or transmission electron microscopy; see for instance Gallier et al. (A novel infant milk formula concept: Mimicking the human milk fat globule structure, Colloids and Surfaces B: Biointerfaces 136 (2015) 329-339).

The nutritional composition preferably comprises glycerophospholipids. Examples of glycerophospholipids are phosphatidylcholine (PC), phosphatidylserine (PS), phosphatidylethanolamine (PE), phosphatidylinositol (PI) and phosphatidylglycerol (PG). Preferably the nutritional composition comprises one or more of PC, PS, PI and PE, more preferably the nutritional composition comprises at least PC.

The nutritional composition preferably comprises sphingomyelin. Sphingomyelins have a phosphorylcholine or phosphorylethanolamine molecule esterified to the 1-hydroxy group of a ceramide. They are classified as phospholipid as well as sphingolipid, but are not classified as a glycerophospholipid nor as a glycosphingolipid. Preferably the nutritional composition comprises 0.05 to 10 wt.% sphingomyelin based on total lipid, more preferably 0.1 to 5 wt.%, even more preferably 0.2 to 2 wt.%. Preferably the nutritional composition comprises at least 5 wt.%, more preferably 5 to 40 wt.% sphingomyelin based on total phospholipid, more preferably 10 to 35 wt.%, even more preferably 15 to 35 wt.%, based on total phospholipid.

The nutritional composition preferably comprises glycosphingolipids. Preferably the nutritional composition comprises 0.1 to 10 wt.% glycosphingolipids based on total lipid, more preferably 0.5 to 5 wt.%, even more preferably 2 to 4 wt.%, based on total lipid. The term glycosphingolipids in the present context particularly refers to glycolipids with an amino alcohol sphingosine. The sphingosine backbone is O-linked to a charged head-group such as ethanolamine, serine or choline backbone. The backbone is also amide linked to a fatty acyl group. Glycosphingolipids are ceramides with one or more sugar residues joined in a beta-glycosidic linkage at the 1-hydroxyl position, and include gangliosides. Preferably the nutritional composition contains gangliosides, more preferably at least one ganglioside selected from the group consisting of GM3 and GD3.

The nutritional composition preferably comprises cholesterol. The nutritional composition preferably comprises at least 0.005 wt.% cholesterol based on total lipid, more preferably at least 0.02 wt.%, more preferably at least 0.05 wt.%., even more preferably at least 0.1 wt.% cholesterol based on total lipid. Preferably the amount of cholesterol does not exceed 10 wt.% based on total lipid, more preferably does not exceed 5 wt.%, even more preferably does not exceed 1 wt.% based on total lipid in the nutritional composition.

Preferred sources for providing the phospholipid, glycosphingolipid and/or cholesterol are egg lipids, milk fat, buttermilk fat and butter serum fat (such as beta serum fat). A preferred source for phospholipid, particularly PC, is soy lecithin and/or sunflower lecithin.

The nutritional composition preferably comprises phospholipid derived from mammalian milk. Preferably the nutritional composition comprises phospholipid and glycosphingolipid derived from mammalian milk. Preferably also cholesterol is obtained from mammalian milk. The nutritional composition preferably comprises phospholipid, glycosphingolipid and/or cholesterol from mammalian milk from cows, mares, sheep, goats, buffalos, horses and/or camels. More preferably the nutritional composition comprises phospholipid, glycosphingolipid and/or cholesterol from cow's milk.

Phospholipid derived from milk includes preferably phospholipid that is isolated from milk lipid, cream lipid, cream serum lipid, butter serum lipid (beta serum lipid), whey lipid, cheese lipid and/or buttermilk lipid. Buttermilk lipid is typically obtained during the manufacture of buttermilk. Butter serum lipid or beta serum lipid is typically obtained during the manufacture of anhydrous milk fat from butter. Preferably the phospholipid, glycosphingolipid and/or cholesterol is obtained from milk cream. Examples of suitable commercially available sources for phospholipid from milk are BAEF, SM2, SM3 and SM4 powder of Corman, Salibra of Glanbia, Lipamin M20 of Lecico and LacProdan MFGM-10 or PL20 of Arla.

The use of phospholipid from milk lipid advantageously comprises the use of milk fat globule membranes, which are more reminiscent to the situation in human milk. The concomitant use of phospholipid derived from milk and triglycerides derived from vegetable lipids therefore enables the manufacture of coated lipid globules with a coating more similar to human milk, while at the same time providing an optimal fatty acid profile.

Preferably the phospholipid is derived from milk lipid, more preferably from cow's milk lipid. Preferably the phospholipid is derived from or forms part of the milk fat globule membrane (MFGM), more preferably is derived from or forms part of cow's MFGM.

Preferably the nutritional composition comprises phospholipid and glycosphingolipid. In a preferred embodiment the weight ratio of phospholipid : glycosphingolipid is from 2:1 to 12:1, more preferably from 2:1 to 10:1 and even more preferably 2:1 to 5:1.

Preferably the nutritional composition comprises phospholipid, glycosphingolipids and cholesterol.

Methods for obtaining lipid globules with an increased size and/or coating with phospholipid are for example disclosed in WO 2010/0027258 and WO 2010/0027259.

### Lipid

The nutritional composition comprises lipid. Lipid in the present invention comprises one or more selected from the group consisting of triglycerides, polar lipids (such as phospholipids, cholesterol, glycolipids, sphingomyelin), free fatty acids, monoglycerides and diglycerides.

The lipid provides preferably 30 to 60 % of the total calories of the nutritional composition. More preferably the nutritional composition comprises lipid providing 35 to 55 % of the total calories, even more preferably the nutritional composition comprises lipids providing 40 to 50 % of the total calories. The lipids are preferably present in an amount of 3 to 7 g per 100 kcal, more preferably in an amount of 4 to 6 g lipid per 100 kcal and most preferably in an amount of 4.5 to 5.5 g lipid per 100 kcal. When in liquid form, e.g. as a ready-to-feed liquid, the nutritional composition preferably comprises 2.1 to 6.5 g lipids per 100 ml, more preferably 3.0 to 4.0 g per 100 ml. Based on dry weight the nutritional composition preferably comprises 10 to 50 wt.%, more preferably 12.5 to 40 wt.% lipids, even more preferably 19 to 30 wt.% lipids.

The lipid preferably comprises vegetable lipids. The presence of vegetable lipids advantageously enables an optimal fatty acid profile high in polyunsaturated fatty acids and/or more reminiscent to human milk fat. Lipid from non-human mammalian milk alone, e.g. cow's milk, does not provide an optimal fatty acid profile. The amount of essential fatty acids is too low in non-human mammalian milk.

Preferably the nutritional composition comprises at least one, preferably at least two vegetable lipid sources selected from the group consisting of linseed oil (flaxseed oil), rape seed oil (such as colza oil, low erucic acid rape seed oil and canola oil), sunflower oil, high oleic sunflower oil, safflower oil, high oleic safflower oil, olive oil, coconut oil, palm oil and palm kernel oil.

In a preferred embodiment, the nutritional composition comprises 5 to 100 wt.% vegetable lipids based on total lipids, more preferably 10 to 95 wt.%, more preferably 20 to 80 wt.%, even more preferably 25 to 75 wt.%, most preferably 40 to 70 wt.% vegetable lipids based on total lipids. It is noted therefore that the nutritional composition also may comprise non-vegetable lipids. Non-vegetable lipids may include mammalian milk fat, mammalian milk derived lipid as a preferred source of phospholipid, and fish, marine and/or microbial oils as source of long chain polyunsaturated fatty acids (LC-PUFA).

### Palmitic acid (PA) at sn-2 position of triglyceride

Triglycerides are the major fraction of the lipids in the nutritional composition. Triglycerides comprise a glycerol moiety to which, via ester bonds, three fatty acid residues are attached, which may be the same or different, and which are generally chosen from saturated and unsaturated fatty acids containing 4 to 26 carbon atoms. Such triglycerides may differ in the fatty acid residues that are present and/or may differ in the respective position(s) of the fatty acid residues to the glycerol backbone (e.g. in the sn-1, sn-2 and/or sn-3 position). Preferably the nutritional composition comprises at least 70 wt.%, more preferably at least 80 wt.%, more preferably at least 85 wt.% triglycerides based on total lipids, even more preferably at least 90 wt.% triglycerides based on total lipids, and most preferably at least 95 wt.% triglycerides based on total lipids.

Lipids that can be used to enhance the amount of PA located at the sn-2 position in triglycerides based on total PA are commercially available - e.g. from Loders Croklaan under the name Betapol^{™} and/or can be prepared in a manner known per se, for instance as described in EP 0698078 and/or EP 0758846. Another suitable source is InFat^{™} of Enzymotec. In case these lipids are obtained by trans- or interesterification of vegetable triglycerides, these sources are in the context of the present invention regarded as vegetable lipids.

A preferred source for triglycerides to enhance PA at the sn-2 or beta position in a triglyceride is non-human animal fat, more preferably non-human mammalian milk fat, even more preferably cow's milk fat. Preferably non-human mammalian milk fat, in particular cow's milk fat, is used in the form of anhydrous milk fat, butter oil, butter fat or butter. Preferably, the source of the milk fat is in a homogenous fat phase, such as butter oil or anhydrous milk fat, and not in the form of oil in water emulsion such as cream, since the lipid globules of the present invention can be more easily prepared during the manufacture of the nutritional composition, when the lipid is added to the aqueous phase as homogenous fat phase, upon which the mixture is treated to form an emulsion.

Preferably the amount of the source of lipid, which comprises triglycerides with an increased amount of palmitic acid residues in the sn-2 position of a triglyceride, that is comprised in the lipid of the nutritional composition, is between 10 and 70 wt.%, more preferably between 20 and 65 wt.%, even more preferably between 30 and 60 wt.% based on total lipid. Such source of lipid is preferably mammalian milk fat, more preferably such source of lipids is non-human mammalian milk fat. The mammalian milk fat is preferably selected from butter, butter fat, butter oil or anhydrous milk fat. Preferably the nutritional composition comprises mammalian milk fat selected from butter, butter fat, butter oil or anhydrous milk fat between 10 and 70 wt.% based on total lipid, more preferably between 20 and 75 wt.%, even more preferably between 30 and 60 wt.% based on total lipid.

In a particularly preferred embodiment, the lipid in the nutritional composition comprises:
a. 30 to 90 wt.% vegetable lipid based on total lipid, and
b. 10 to 70 wt.% mammalian milk fat based on total lipid, wherein the mammalian milk fat is selected from butter, butter fat, butter oil or anhydrous milk fat.

More preferably, the lipid in the nutritional composition comprises:
a. 35 to 80 wt.% vegetable lipid based on total lipid, and
b. 20 to 65 wt.% mammalian milk fat based on total lipid, wherein the mammalian milk fat is selected from butter, butter fat, butter oil or anhydrous milk fat.

Most preferably, the lipid in the nutritional composition comprises:
a. 40 to 70 wt.% vegetable lipid based on total lipid, and
b. 30 to 60 wt.% mammalian milk fat based on total lipid, wherein the mammalian milk fat is selected from butter, butter fat, butter oil or anhydrous milk fat.

In a preferred embodiment, the lipid comprises at least 10 wt.% palmitic acid (PA) based on total fatty acids and at least 15 wt.% of palmitic acid, based on total palmitic acid, is located at the sn-2 position of a triglyceride.

The lipids in the nutritional composition are preferably chosen such that the amount of palmitic acid (PA) that is present in the total lipid of the nutritional composition is at least 10 wt.% based on total fatty acids in the total lipid, preferably at least 15 wt.%. Preferably the amount of PA that is present in the total lipid is below 30 wt.% based on total fatty acids. More preferably the amount of PA that is present in the lipid is from 12 to 26 wt.% based on total fatty acids in the total lipid, even more preferably from 14 to 24 wt.%, even more preferably from 16 to 22 wt.%.

The lipids in the nutritional composition are preferably chosen such that, based on the total PA present in the lipid, at least 15 wt.%, preferably at least 20 wt.%, more preferably at least 25 wt.%, more preferably at least 30 wt.% PA is in the sn-2 or beta position in a triglyceride. Preferably the amount of PA in the sn-2 position in a triglyceride is not more than 45 wt.%, preferably not more than 40 wt.% based on total PA present in the lipid. Preferably the amount of PA in the sn-2 position in a triglyceride is from 25 to 40 wt.% based on total PA present in the total lipid.

Lipid in the form of large lipid globules and comprising a coating comprising phospholipid, together with the presence of an enhanced amount of sn-2 palmitic acid or with the presence of mammalian milk fat, showed a further improvement of the reduction of the risk of developing NAFLD in infants at risk of developing NAFLD.

### Fatty acid composition

SFA relates to saturated fatty acids and/or acyl chains, MUFA relates to mono-unsaturated fatty acid and/or acyl chains, PUFA refers to polyunsaturated fatty acids and/or acyl chains with 2 or more unsaturated bonds; LC-PUFA refers to long chain polyunsaturated fatty acids and/or acyl chains comprising at least 20 carbon atoms in the fatty acyl chain and with 2 or more unsaturated bonds; Medium chain fatty acids (MCFA) refer to fatty acids and/or acyl chains with a chain length of 6, 8 or 10 carbon atoms. n3 or omega-3 PUFA refers to polyunsaturated fatty acids and/or acyl chains with 2 or more unsaturated bonds with an unsaturated bond at the third carbon atom from the methyl end of the fatty acyl chain, n6 or omega-6 PUFA refers to polyunsaturated fatty acids and/or acyl chains with 2 or more unsaturated bonds with an unsaturated bond at the sixth carbon atom from the methyl end of the fatty acyl chain.

DHA refers to docosahexaenoic acid and/or acyl chain (22:6, n3); DPA refers to docosapentaenoic acid and/or acyl chain (22:5 n3). EPA refers to eicosapentaenoic acid and/or acyl chain (20:5 n3); ARA refers to arachidonic acid and/or acyl chain (20:4 n6). LA refers to linoleic acid and/or acyl chain (18:2 n6); ALA refers to alpha-linolenic acid and/or acyl chain (18:3 n3). PA relates to palmitic acid and/or acyl chains (C16:0). BA refers to butyric acid (4:0).

The nutritional composition according to the present use preferably comprises LA. LA is an n6 PUFA and the precursor of n6 LC-PUFA and is an essential fatty acid as it cannot be synthesized by the human body. LA preferably is present in a sufficient amount in order to promote a healthy growth and development, yet in an amount as low as possible to prevent negative, competitive, effects on the formation of n3 PUFA and a too high n6/n3 ratio. The nutritional composition therefore preferably comprises less than 15 wt.%, more preferably less than 12 wt.%, more preferably less than 10 wt.% LA based on total fatty acids. The nutritional composition preferably comprises at least 5 wt.% LA based on fatty acids, preferably at least 6 wt.% LA, more preferably at least 7 wt.% LA based on total fatty acids.

The nutritional composition preferably comprises ALA. ALA is a n3 PUFA and the precursor of n3 LC-PUFA and is an essential fatty acid as it cannot be synthesized by the human body. Preferably ALA is present in a sufficient amount to promote a healthy growth and development of the infant. The nutritional composition therefore preferably comprises at least 1.0 wt.%, more preferably the nutritional composition comprises at least 1.5 wt.%, even more preferably at least 2.0 wt.% ALA based on total fatty acids. Preferably the nutritional composition comprises less than 10 wt.% ALA, more preferably less than 5.0 wt.% based on total fatty acids.

The weight ratio LA/ALA preferably is well balanced in order to ensure an optimal n6/n3 PUFA, n6/n3 LC PUFA and DHA/ARA ratio in the cellular membranes. Therefore, the nutritional composition preferably comprises a weight ratio of LA/ALA from 2 to 20, more preferably from 3 to 15, more preferably from 5 to 12, more preferably from 5 to 10. Preferably the n6 PUFA/n3 PUFA weight ratio is from 3 to 20, more preferably from 3 to 15, more preferably from 5 to 12, more preferably from 5 to 10.

Preferably, the nutritional composition comprises n3 LC-PUFA, such as EPA, DPA and/or DHA, more preferably DHA. As the conversion of ALA to DHA may be less efficient in infants, preferably both ALA and DHA are present in the nutritional composition. Preferably the nutritional composition comprises at least 0.05 wt.%, preferably at least 0.1 wt.%, more preferably at least 0.2 wt.%, of DHA based on total fatty acids. Preferably the nutritional composition comprises not more than 2.0 wt.%, preferably not more than 1.0 wt.% of DHA based on total fatty acids.

The nutritional composition preferably comprises ARA. Preferably the nutritional composition comprises at least 0.05 wt.%, preferably at least 0.1 wt.%, more preferably at least 0.2 wt.% of ARA based on total fatty acids. As the group of n6 fatty acids, especially arachidonic acid (ARA) counteracts the group of n3 fatty acids, especially DHA, the nutritional composition preferably comprises relatively low amounts of ARA. Preferably the nutritional composition comprises not more than 2.0 wt.%, preferably not more than 1.0 wt.% of ARA based on total fatty acids. Preferably the weight ratio between DHA and ARA is between 1:4 to 4:1, more preferably between 1:2 to 2:1, more preferably between 0.6 and 1.5.

### Digestible carbohydrates

The nutritional composition comprises digestible carbohydrates. The digestible carbohydrates preferably provide 25 to 75% of the total calories of the nutritional composition. Preferably the digestible carbohydrates provide 40 to 60% of the total calories. Based on calories the nutritional composition preferably comprises of 5 to 20 g of digestible carbohydrates per 100 kcal, more preferably 6 to 16 g per 100 kcal. When in liquid form, e.g. as a ready-to-feed liquid, the nutritional composition preferably comprises 3 to 30 g digestible carbohydrate per 100 ml, more preferably 6 to 20, even more preferably 7 to 10 g per 100 ml. Based on dry weight the nutritional composition preferably comprises 20 to 80 wt.%, more preferably 40 to 65 wt.% digestible carbohydrates.

Preferred digestible carbohydrate sources are lactose, glucose, sucrose, fructose, galactose, maltose, starch and maltodextrin. Lactose is the main digestible carbohydrate present in human milk. Lactose advantageously has a low glycaemic index. The nutritional composition preferably comprises lactose. The nutritional composition preferably comprises digestible carbohydrate, wherein at least 35 wt.%, more preferably at least 50 wt.%, more preferably at least 75 wt.%, even more preferably at least 90 wt.%, most preferably at least 95 wt.% of the digestible carbohydrate is lactose. Based on dry weight the nutritional composition preferably comprises at least 25 wt.% lactose, preferably at least 40 wt.% lactose.

### Protein

The nutritional composition comprises protein. The protein preferably provides 5 to 20% of the total calories. Preferably the nutritional composition comprises protein that provides 6 to 12% of the total calories. Preferably the nutritional composition comprises less than 3.5 g protein per 100 kcal, more preferably the nutritional composition comprises between 1.5 and 2.1 g protein per 100 kcal, even more preferably between 1.6 and 2.0 g protein per 100 kcal. A low protein concentration advantageously is closer to human milk as human milk comprises a lower amount of protein based on total calories compared to cow's milk. The protein concentration in a nutritional composition is determined by the sum of protein, peptides and free amino acids. Based on dry weight the nutritional composition preferably comprises less than 12 wt.% protein, more preferably between 9.6 and 12 wt.%, even more preferably between 10 and 11 wt.%. Based on a ready-to-drink liquid product the nutritional composition preferably comprises less than 1.5 g protein per 100 ml, more preferably between 1.2 and 1.5 g, even more preferably between 1.25 and 1.35 g per 100 ml.

The source of the protein should be selected in such a way that the minimum requirements for essential amino acid content are met and satisfactory growth is ensured. Hence protein sources based on cows' milk proteins such as whey, casein and mixtures thereof and proteins based on soy, potato or pea are preferred. In case whey proteins are used, the protein source is preferably based on acid whey or sweet whey, whey protein isolate or mixtures thereof. Preferably the nutritional composition comprises at least 3 wt.% casein based on dry weight. Preferably the casein is intact and/or non-hydrolyzed.

### Non-digestible carbohydrates

In one embodiment the nutritional composition preferably comprises non-digestible oligosaccharides. Preferably the nutritional composition comprises non-digestible oligosaccharides with a degree of polymerization (DP) between 2 and 250, more preferably between 3 and 60.

Preferably the nutritional composition comprises fructo-oligosaccharides, galacto-oligosaccharides and/or galacturonic acid oligosaccharides, more preferably fructo-oligosaccharides and/or galacto-oligosaccharides, even more preferably galacto-oligosaccharides, most preferably transgalactooligosaccharides. In a preferred embodiment the nutritional composition comprises a mixture of galacto-oligosaccharides and fructo-oligosaccharides, more preferably transgalactooligosaccharides and fructo-oligosaccharides. Suitable non-digestible oligosaccharides are for example Vivinal^{®}GOS (FrieslandCampina DOMO), Raftilin^{®}HP or Raftilose^{®} (Orafti).

Preferably, the nutritional composition comprises 80 mg to 2 g non-digestible oligosaccharides per 100 ml, more preferably 150 mg to 1.5 g, even more preferably 300 mg to 1 g per 100 ml. Based on dry weight, the nutritional composition preferably comprises 0.25 wt.% to 20 wt.%, more preferably 0.5 wt.% to 10 wt.%, even more preferably 1.5 wt.% to 7.5 wt.% of non-digestible oligosaccharides.

### Formula

The use according to the present invention requires the administration of an infant formula, a follow-on formula or a growing-up milk. This means that the composition that is administered is not human milk. It also means that the composition that is administered is not native cow's milk or native milk from another mammal. Alternatively, the terms as used herein, "infant formula" or "follow-on formula" or "growing-up milk" means that it concerns a composition that is artificially made or in other words that it is synthetic. Hence in one embodiment, the nutritional composition that is administered is an artificial infant formula or an artificial follow-on formula or an artificial growing-up milk or a synthetic infant formula or a synthetic follow-on formula or a synthetic growing-up milk.

In the present context, infant formula refers to nutritional compositions, artificially made, intended for infants of 0 to about 4 to 6 months of age and are intended as a substitute for human milk. Typically, infant formulae are suitable to be used as sole source of nutrition. Such infant formulae are also known as starter formula. Follow-on formula for infants starting with at 4 to 6 months of life to 12 months of life are intended to be supplementary feedings to infants that start weaning on other foods. Infant formulae and follow-on formulae are subject to strict regulations, for example for the EU regulations no. 609/2013 and no. 2016/127. In the present context, growing-up milk refers to nutritional compositions, artificially made, intended for infants of 12 months to 36 months, which are intended to be supplementary feedings to infants.

The nutritional composition is preferably an infant formula or a follow-on formula. More preferably the nutritional composition is an infant formula.

The nutritional composition is preferably an infant formula or follow-on formula and preferably comprises 3 to 7 g lipid/100 kcal, preferably 4 to 6 g lipid/100 kcal, more preferably 4.5 to 5.5 g lipid/100 kcal, preferably comprises 1.7 to 3.5 g protein/100 kcal, more preferably 1.8 to 2.1 g protein/100 kcal, more preferably 1.8 to 2.0 g protein/100 kcal and preferably comprises 5 to 20 g digestible carbohydrate/100 kcal, preferably 6 to 16 g digestible carbohydrate/100 kcal, more preferably 10 to 15 g digestible carbohydrate/100 kcal.

Preferably the nutritional composition is an infant formula or follow-on formula, and preferably has an energy density of 60 kcal to 75 kcal/100 ml, more preferably 60 to 70 kcal/100 ml, when in a ready-to-drink form. This density ensures an optimal balance between hydration and caloric intake.

In one embodiment, the nutritional composition is a powder. Suitably, the nutritional composition is in a powdered form, which can be reconstituted with water or other food grade aqueous liquid, to form a ready-to drink liquid, or is in a liquid concentrate form that should be diluted with water to a ready-to-drink liquid. It was found that lipid globules maintained their size and coating when reconstituted.

In this document and in its claims, the verb "to comprise" and its conjugations is used in its nonlimiting sense to mean that items following the word are included, but items not specifically mentioned are not excluded. In addition, reference to an element by the indefinite article "a" or "an" does not exclude the possibility that more than one of the element is present, unless the context clearly requires that there be one and only one of the elements. The indefinite article "a" or "an" thus usually means "at least one".

### EXAMPLES

### Example 1: Effect of lipid globule design on hepatic triglyceride levels in offspring of dams with gestational diabetes

An experiment was performed wherein the effects of an infant milk formula (IMF) with large phospholipid coated lipid globules (IMF 1) was compared with a control IMF with small lipid globules and no phospholipid coating (IMF 2).

### GDM mouse model

A gestational diabetes mellitus (GDM) mouse model was used to generate offspring which was exposed to GDM conditions during gestation. The dams received 2 triggers before pregnancy in order to induce a transient GDM condition. This approach reflected the GDM pathophysiology in a pilot study (data not shown).

At 12 weeks of age C57BL/6N dams were fed with a 60 energy % high fat diet (HFD) for 4 weeks to reduce insulin sensitivity. The HFD was based on AIN93G diet with an adjusted lipid fraction containing 60 energy % lipid. A low dose of Streptozotocin (60 mg STZ/kg) was administered on 3 consecutive days prior to mating. STZ is an alkylating agent that selectively kills pancreatic betacells via cell necrosis and/or apoptosis. By administrating low doses of STZ, the beta-cell capacity is reduced. The HFD feeding was continued until weaning at post-natal day 16 (PN16). The offspring was breastfed/lactated with breast milk till PN16. At PN16, the male offspring was weaned and administered with IMF 1 (n=9) or the control IMF 2 (n=8) until PN42. The offspring was subsequently fed with Western-style diet (WSD) for 15 weeks. The WSD was based on AIN93G diet with an adjusted lipid fraction containing 45 energy% lipid.

At PN147, the mall offspring were fasted for 4 hours and subsequently sacrificed. The livers were collected, partially split in Trizol, snap-frozen in liquid nitrogen, and then stored at -80°C until further analysis. The liver samples were homogenized on ice. Liver lipid was extracted according to the procedure of Bligh & Dyer (dichloromethane / methanol extraction). Hepatic triglyceride (TG) content was determined by GPO-PAP enzymatic assay (Trig/GB kit, Roche, 11877771, Mannheim, Germany). In order to normalize the hepatic lipid content to protein, a protein assay adapted from Lowry method was performed. Also, hepatic fatty acid (FA) profile was quantified by means of gas chromatographic analysis. The principle of fatty acids profile analysis is by the trans-methylation of fatty acids from various biological samples and by measurement of fatty acids relatively to the nonindigenous fatty acid standard (C17:0) using gas chromatography [F. A. Muskiet et al., J. Chromatogr. 278, 231-244 (1983)].

### Diets

The offspring diets comprised a macronutrient and micronutrient composition following AIN93G. The offspring diets consisted of 28.3% (w/w) IMF 1 or control IMF 2. Protein, carbohydrates, and micronutrients were added to match AIN93G. The fat components were derived entirely from the IMF. The fat content and fatty acid profile of the diets comprising IMF 1 and IMF 2 were similar (Table 1).

For IMF 1 a mix of anhydrous cow's milk fat, coconut oil, low erucic acid rape seed oil, sunflower oil, high oleic acid sunflower oil, with a small amount of soy lecithin and LC-PUFA premix was used. In addition, IMF 1 comprised butter serum powder as a source of milk derived phospholipids. The amount of vegetable lipid was about 53 wt.% based on total fat, and the amount of mammalian milk fat about 47 wt.%.

For control IMF 2 a mixture of palm oil, coconut oil, low erucic acid rape seed oil, sunflower oil, high oleic sunflower oil, with a small amount of soy lecithin, and LC-PUFA premix was used. The amount of vegetable lipid in the final control IMF was about 98 wt.% based on total fat, and the amount of mammalian milk fat about 1 wt.%.

**Table 1**

| | IMF 1 | IMF 2 |
|---|---|---|
| Energy percentage (%) | | |
| Protein | 19.2 | 19.2 |
| Carbohydrate | 64.4 | 64.4 |
| Fat | 16.4 | 16.4 |
| | | |

| Fatty acid composition (g/kg) | | |
|---|---|---|
| Saturated fatty acids | 28.9 | 28.6 |
| Monounsaturated fatty acids | 26.1 | 26.3 |
| Linoleic acid | 9.1 | 9.3 |
| Alpha linolenic acid | 1.7 | 1.7 |
| Arachidonic acid | 0.2 | 0.2 |
| Docosahexaenoic acid | 0.13 | 0.13 |
| Total omega-6 PUFA | 9.4 | 9.5 |
| Total omega-3 PUFA | 1.9 | 1.9 |
| | | |
| Cholesterol (mg/kg) | 21.7 | 4.8 |

IMF 1 was prepared in a similar way as described in example 1B of WO 2010/0027259. The lipid globules of IMF 1 were large and coated with phospholipids. The control IMF 2 was prepared with high pressure homogenization, resulting in small lipid globules.

The detailed characteristics of the fat component of the different experimental IMFs used for the different IMFs are shown in Table 2.

**Table 2**

| | IMF 1 | IMF 2 |
|---|---|---|
| PA (wt.% based on total FA) | 19.2 | 18.3 |
| % PA at sn-2/total PA | 35.1 | 11.4 |
| PL (wt.% based on total lipid) | 2.37 | 0.13 |
| Milk derived PL (wt.% based on total lipid) | 2.34 | 0 |
| Mode diameter based on volume | Between 3-5 µm | Between 0.3-0.5 µm |
| Volume-based percentage of lipid globules having a diameter between 2 and 12 µm | ≥ 45% | <20% |

### Results

At PN147 the bodyweight and adiposity was measured. After a long exposure to WSD no statistically significant differences in body weight and adiposity were observed between the fasted male mice that were fed IMF 1 or IMF 2.

The levels of TG and FA in the liver was determined. Table 3 shows the measured levels of TG and FA.

**Table 3**

| | IMF 1 (mean + SEM) | IMF 2 (mean + SEM) |
|---|---|---|
| Hepatic TG (nmol/mg protein) | 78 (9) * | 187 (22) |
| Total FA µmol-/gram liver) | 154.4 (16.7) * | 343.5 (33.4) |
| SFA µmol-/gram liver) | 50.5 (5.4) * | 115.7 (11.1) |
| UFA µmol-/gram liver) | 103.9 (11.5) * | 227.8 (22.4) |
| MFA µmol-/gram liver) | 57.9 (9.8) * | 156.8 (18.2) |
| PUFA µmol-/gram liver) | 46.0 (2.0) * | 71.0 (4.9) |

| | | |
|---|---|---|
| * p-value < 0.05 IMF 1 vs. IMF 2, using a Mann-Whitney test. | | |

As can be deduced from Table 3, the levels of hepatic TG and hepatic total FA in mice which consumed IMF 1 in early life and subsequently have been exposed to a WSD diets were significantly different lower compared to the levels of hepatic TG and total hepatic FA in the mice that consumed IMF 2 in early life. Lower levels of TG and lower levers of total FA are considered healthier and are indicative of a reduced risk of developing non-alcoholic fatty liver disease in the mice that were fed IMF1 early in life.

Since the energy intake was very similar for both offspring groups, it is surprising that the difference in lipid globule design (larger lipid globules with PL coating) in an IMF fed early in life has such a protective effect against excessive fat storage in the liver when challenged later in life.

## Claims

1. A nutritional composition, selected from infant formula, follow-on formula and growing-up milk, for use in reducing the risk of developing non-alcoholic fatty liver disease (NAFLD) in a human subject selected from the group consisting of:
a. an infant born to an overweight and/or obese mother at the time of conception of the infant;
b. an infant born to a mother with diabetes at the time of conception of the infant;
c. an infant born to a mother with gestational diabetes (GDM); and
d. an infant who was large for its gestational age (LGA) at birth;
said nutritional composition comprising digestible carbohydrates, protein and lipid, wherein the lipid is in the form of lipid globules and wherein:
i. the lipid globules have a mode diameter, based on volume, of at least 1.0 µm and/or at least 45 volume %, based on total lipid volume, of the lipid globules have a diameter of 2 to 12 µm; and
ii. the lipid globules are at least partly coated on the surface with phospholipids, the amount of phospholipids present in the nutritional composition being from 0.5 to 20 wt.% phospholipids based on total lipids.

2. The nutritional composition for use according to claim 1, wherein the human subject is selected from the group consisting of:
a. an infant born to an overweight and/or obese mother at the time of conception of the infant;
b. an infant born to a mother with diabetes at the time of conception of the infant; and
c. an infant born to a mother with GDM.

3. The nutritional composition for use according to claim 1 or 2, wherein the human subject is selected from the group consisting of:
a. an infant born to a mother with diabetes at the time of conception of the infant; and
b. an infant born to a mother with GDM;

4. The nutritional composition for use according to any of the preceding claims, wherein the human subject is an infant born to a mother with GDM.

5. The nutritional composition for use according to any of the preceding claims, wherein the human subject is an infant born to an overweight and/or obese mother at the time of conception of the infant and wherein the mother also has GDM.

6. The nutritional composition for use according to any of the preceding claims for reducing the risk of developing paediatric NAFLD.

7. The nutritional composition for use according to any of the preceding claims, wherein the risk of developing NAFLD is reduced compared to the risk of developing NAFLD in a human subject selected from the group consisting of:
a. an infant born to an overweight and/or obese mother at the time of conception of the infant;
b. an infant born to a mother with diabetes at the time of conception of the infant;
c. an infant born to a mother with GDM; and
d. an infant who was LGA at birth;
wherein the subject was fed a nutritional composition, selected from infant formula, follow-on formula and growing-up milk, comprising digestible carbohydrates, protein and lipid, said nutritional composition comprising less than 0.5 wt.% phospholipids based on total lipids and having lipid globules with a mode diameter, based on volume, of about 0.5 µm.

8. The nutritional composition for use according to any of the preceding claims, wherein the lipid further comprises at least 10 wt.% palmitic acid based on total fatty acids and at least 15 wt.% of palmitic acid, based on total palmitic acid, is located at the sn-2 position of a triglyceride.

9. The nutritional composition for use according to any of the preceding claims, wherein the lipid in the nutritional composition comprises:
∘ 30 to 90 wt.% vegetable lipid based on total lipid; and
∘ 10 to 70 wt.% mammalian milk fat based on total lipid, wherein the mammalian milk fat is selected from butter, butter fat, butter oil and anhydrous milk fat.

10. The nutritional composition for use according to any of the preceding claims, wherein the phospholipids are derived from or form part of the milk fat globule membrane (MFGM), preferably cow's milk MFGM.

11. The nutritional composition for use according to any one of the preceding claims, wherein the phospholipids are derived from mammalian milk.

12. The nutritional composition for use according to any of the preceding claims comprising less than 15 wt.% linoleic acid and more than 1 wt.% alpha-linolenic acid based on total fatty acids.

13. The nutritional composition for use according to any of the preceding claims wherein the lipids provide 30 to 60 % of the total calories, the protein provides 5 to 20% of the total calories and the digestible carbohydrates provide 25 to 75% of the total calories.

14. The nutritional composition for use according to any one of the preceding claims, wherein the composition is a powder, suitable to reconstitute with water to a ready-to-drink formula.

15. The nutritional composition for use according to any of the preceding claims, wherein the nutritional composition is selected from an infant formula and a follow-on formula, preferably is an infant formula.

## Patentansprüche

1. Ernährungszusammensetzung, die aus Säuglingsnahrung, Folgenahrung oder Kleinkindmilch ausgewählt wird, zur Verwendung bei der Verringerung des Risikos der Entwicklung einer nichtalkoholischen Fettlebererkrankung (NAFLD) bei einem menschlichen Patienten, ausgewählt aus der Gruppe, die aus Folgendem besteht:
a. einem Säugling, der von einer übergewichtigen und/oder fettleibigen Mutter zum Zeitpunkt der Empfängnis des Säuglings geboren wurde;
b. einem Säugling, der von einer Mutter geboren wurde, die zum Zeitpunkt der Empfängnis des Säuglings an Diabetes erkrankt war;
c. einem Säugling, der von einer Mutter mit Schwangerschaftsdiabetes (GDM) geboren wurde; und
d. einem Säugling, der bei der Geburt für sein Gestationsalter (LGA) groß war; wobei die Ernährungszusammensetzung verdauliche Kohlenhydrate, Protein und Lipid umfasst, wobei das Lipid in Form von Lipidkügelchen vorliegt und wobei:
i. die Lipidkügelchen einen Modus-Durchmesser, bezogen auf das Volumen, von mindestens 1,0 µm haben und/oder mindestens 45 Volumen%, bezogen auf das Gesamtlipidvolumen, der Lipidkügelchen einen Durchmesser von 2 bis 12 µm haben; und
ii. die Lipidkügelchen zumindest teilweise auf der Oberfläche mit Phospholipiden beschichtet sind, wobei die Menge der in der Ernährungszusammensetzung vorhandenen Phospholipide 0,5 bis 20 Gew.-% Phospholipide, bezogen auf die Gesamtlipide, beträgt.

2. Ernährungszusammensetzung zur Verwendung nach Anspruch 1, wobei der menschliche Patient aus der Gruppe, die aus Folgendem besteht, ausgewählt wird:
a. einem Säugling, der von einer übergewichtigen und/oder fettleibigen Mutter zum Zeitpunkt der Empfängnis des Säuglings geboren wurde;
b. einem Säugling, der von einer Mutter geboren wurde, die zum Zeitpunkt der Empfängnis des Säuglings an Diabetes erkrankt war; und
c. einem Säugling, der von einer Mutter mit GDM geboren wurde.

3. Ernährungszusammensetzung zur Verwendung nach Anspruch 1 oder 2, wobei der menschliche Patient aus der Gruppe, die aus Folgendem besteht, ausgewählt wird:
a. einem Säugling, der von einer Mutter geboren wurde, die zum Zeitpunkt der Empfängnis des Säuglings an Diabetes erkrankt war; und
b. einem Säugling, der von einer Mutter mit GDM geboren wurde;

4. Ernährungszusammensetzung zur Verwendung nach einem der vorhergehenden Ansprüche, wobei der menschliche Patient ein Säugling ist, der von einer Mutter mit GDM geboren wurde.

5. Ernährungszusammensetzung zur Verwendung nach einem der vorhergehenden Ansprüche, wobei der menschliche Patient ein Säugling ist, der von einer übergewichtigen und/oder fettleibigen Mutter zum Zeitpunkt der Empfängnis des Säuglings geboren wurde und wobei die Mutter auch GDM hat.

6. Ernährungszusammensetzung zur Verwendung nach einem der vorhergehenden Ansprüche zur Verringerung des Risikos der Entwicklung einer pädiatrischen NAFLD.

7. Ernährungszusammensetzung zur Verwendung nach einem der vorhergehenden Ansprüche, wobei das Risiko der Entwicklung von NAFLD im Vergleich zum Risiko der Entwicklung von NAFLD, bei einem menschlichen Patienten, ausgewählt aus der Gruppe, die aus Folgendem besteht, reduziert ist:
a. einem Säugling, der von einer übergewichtigen und/oder fettleibigen Mutter zum Zeitpunkt der Empfängnis des Säuglings geboren wurde;
b. einem Säugling, der von einer Mutter geboren wurde, die zum Zeitpunkt der Empfängnis des Säuglings an Diabetes erkrankt war;
c. einem Säugling, der von einer Mutter mit GDM geboren wurde; und
d. einem Säugling, der bei der Geburt LGA hatte;
wobei der Patient mit einer Ernährungszusammensetzung gefüttert wurde, die aus Säuglingsnahrung, Folgenahrung und Kleinkindmilch ausgewählt wird, und verdauliche Kohlenhydrate, Proteine und Lipide umfasst, wobei die Ernährungszusammensetzung weniger als 0,5 Gew.-% Phospholipide, bezogen auf die Gesamtlipide umfasst und Lipidkügelchen mit einem Modus-Durchmesser, bezogen auf das Volumen, von etwa 0,5 µm aufweist.

8. Ernährungszusammensetzung zur Verwendung nach einem der vorhergehenden Ansprüche, wobei das Lipid ferner mindestens 10 Gew.-% Palmitinsäure, bezogen auf die Gesamtfettsäuren, umfasst und mindestens 15 Gew.-% der Palmitinsäure, bezogen auf die Gesamtpalmitinsäure, an der sn-2-Position eines Triglycerids angeordnet sind.

9. Ernährungszusammensetzung zur Verwendung nach einem der vorhergehenden Ansprüche, wobei das Lipid in der Ernährungszusammensetzung Folgendes umfasst:
o 30 bis 90 Gew.-% pflanzliches Lipid, bezogen auf das Gesamtlipid; und
o 10 bis 70 Gew.-% Säugetiermilchfett, bezogen auf das Gesamtlipid, wobei das Säugetiermilchfett aus Butter, Butterfett, Butteröl und wasserfreiem Milchfett ausgewählt ist.

10. Ernährungszusammensetzung zur Verwendung nach einem der vorhergehenden Ansprüche, wobei die Phospholipide von der Milchfettkügelchenmembran (MFGM), vorzugsweise Kuhmilch-MFGM, abgeleitet sind oder einen Teil davon bilden.

11. Ernährungszusammensetzung zur Verwendung nach einem der vorhergehenden Ansprüche, wobei die Phospholipide aus Säugetiermilch stammen.

12. Ernährungszusammensetzung zur Verwendung nach einem der vorhergehenden Ansprüche, die weniger als 15 Gew.-% Linolsäure und mehr als 1 Gew.-% Alpha-Linolensäure, bezogen auf die Gesamtfettsäuren, umfasst.

13. Ernährungszusammensetzung zur Verwendung nach einem der vorhergehenden Ansprüche, wobei die Lipide 30 bis 60 % der Gesamtkalorien liefern, das Protein 5 bis 20 % der Gesamtkalorien liefert und die verdaulichen Kohlenhydrate 25 bis 75 % der Gesamtkalorien liefern.

14. Ernährungszusammensetzung zur Verwendung nach einem der vorhergehenden Ansprüche, wobei die Zusammensetzung ein Pulver ist, das mit Wasser zu einer trinkfertigen Formel rekonstituiert werden kann.

15. Ernährungszusammensetzung zur Verwendung nach einem der vorhergehenden Ansprüche, wobei die Ernährungszusammensetzung aus einer Säuglingsnahrung und einer Folgenahrung ausgewählt ist, vorzugsweise eine Säuglingsnahrung ist.

## Revendications

1. Composition nutritionnelle, choisie parmi les formules pour nourrissons, les formules de suite et le lait de croissance, pour utilisation permettant de réduire le risque de développer une stéatose hépatique d'origine non-alcoolique (NAFLD) chez un sujet humain choisi dans le groupe consistant en :
a. un nourrisson né d'une mère en surpoids et/ou obèse au moment de la conception du nourrisson ;
b. un nourrisson né d'une mère diabétique au moment de la conception du nourrisson ;
c. un nourrisson né d'une mère atteinte de diabète gestationnel (GDM) ; et
d. un nourrisson qui était grand pour son âge gestationnel (LGA) à la naissance ;
ladite composition nutritionnelle comprenant des carbohydrates digestibles, des protéines et des lipides, où les lipides se présentent sous la forme de globules lipidiques et où :
i. les globules lipidiques ont un diamètre de mode, sur la base du volume, d'au moins 1,0 µm et/ou au moins 45 % en volume, sur la base du volume de lipide total, des globules lipidiques ont un diamètre de 2 à 12 µm ; et
ii. les globules lipidiques sont au moins partiellement recouverts en surface de phospholipides, la quantité de phospholipides présente dans la composition nutritionnelle étant comprise entre 0,5 et 20 % en poids de phospholipides sur la base des lipides totaux.

2. Composition nutritionnelle pour utilisation selon la revendication 1, où le sujet humain est choisi parmi le groupe consistant en :
a. un nourrisson né d'une mère en surpoids et/ou obèse au moment de la conception du nourrisson ;
b. un nourrisson né d'une mère diabétique au moment de la conception du nourrisson ;
c. un nourrisson né d'une mère atteinte de GDM.

3. Composition nutritionnelle pour utilisation selon la revendication 1 ou 2, où le sujet humain est choisi parmi le groupe consistant en :
a. un nourrisson né d'une mère diabétique au moment de la conception du nourrisson ; et
b. un nourrisson né d'une mère atteinte de GDM ;

4. Composition nutritionnelle pour utilisation selon l'une quelconque des revendications précédentes, où le sujet humain est un nourrisson né d'une mère atteinte de GDM.

5. Composition nutritionnelle pour utilisation selon l'une quelconque des revendications précédentes, où le sujet humain est un nourrisson né d'une mère en surpoids et/ou obèse au moment de la conception du nourrisson et où la mère est également atteinte de GDM.

6. Composition nutritionnelle pour utilisation selon l'une quelconque des revendications précédentes permettant de réduire le risque de développer une NAFLD pédiatrique.

7. Composition nutritionnelle pour utilisation selon l'une quelconque des revendications précédentes, où le risque de développer la NAFLD est réduit par rapport au risque de développer la NAFLD chez un sujet humain choisi dans le groupe consistant en :
a. un nourrisson né d'une mère en surpoids et/ou obèse au moment de la conception du nourrisson ;
b. un nourrisson né d'une mère diabétique au moment de la conception du nourrisson ;
c. un nourrisson né d'une mère atteinte de GDM ; et
d. un nourrisson qui était LGA à la naissance ;
où le sujet a été nourri avec une composition nutritionnelle, choisie parmi les formules pour nourrissons, les formules de suite et le lait de croissance, comprenant des carbohydrates digestibles, des protéines et des lipides, ladite composition nutritionnelle comprenant moins de 0,5 % en poids de phospholipides sur la base des lipides totaux et ayant des globules lipidiques avec un diamètre de mode, sur la base de volume, d'environ 0,5 µm.

8. Composition nutritionnelle pour utilisation selon l'une quelconque des revendications précédentes, où le lipide comprend en outre au moins 10 % en poids d'acide palmitique sur la base des acides gras totaux et au moins 15 % en poids d'acide palmitique, sur la base de l'acide palmitique totale, est situé à la position sn-2 d'un triglycéride.

9. Composition nutritionnelle pour utilisation selon l'une quelconque des revendications précédentes, où le lipide dans la composition nutritionnelle comprend :
o 30 à 90 % en poids de lipides végétaux sur la base des lipides totaux ; et
o 10 à 70 % en poids de graisse de lait de mammifère sur la base des lipides totaux, où la graisse de lait de mammifère est choisie parmi du beurre, la graisse de beurre, l'huile de beurre et la graisse de lait anhydre.

10. Composition nutritionnelle pour utilisation selon l'une quelconque des revendications précédentes, où les phospholipides sont dérivés de ou font partie de la membrane de globules de la graisse de lait (MFGM), de préférence la MFGM du lait de vache.

11. Composition nutritionnelle pour utilisation selon l'une quelconque des revendications précédentes, où les phospholipides sont dérivés de lait de mammifère.

12. Composition nutritionnelle pour utilisation selon l'une quelconque des revendications précédentes comprenant moins de 15 % en poids d'acide linoléique et plus de 1 % en poids d'acide alpha-linolénique sur la base des acides gras totaux.

13. Composition nutritionnelle pour utilisation selon l'une quelconque des revendications précédentes où les lipides fournissent 30 à 60 % des calories totales, les protéines fournissent 5 à 20% des calories totales et les carbohydrates digestibles fournissent 25 à 75% des calories totales.

14. Composition nutritionnelle pour utilisation selon l'une quelconque des revendications précédentes, où la composition est une poudre, apte à être reconstituée avec de l'eau en une formule prête à boire.

15. Composition nutritionnelle pour utilisation selon l'une quelconque des revendications précédentes, où la composition nutritionnelle est choisie parmi une formule pour nourrissons et une formule de suite, de préférence est une formule pour nourrissons.
